(19) Europäisches Patentamt
European Patent Office
Office européen des brevets

(11) Veröffentlichungsnummer: **0 552 651 A2**

(12) # EUROPÄISCHE PATENTANMELDUNG

(21) Anmeldenummer: **93100374.3**

(22) Anmeldetag: **13.01.93**

(51) Int. Cl.5: **C07D 411/04**, C07D 497/04, C09K 15/12, //(C07D497/04, 327:00,307:00)

(30) Priorität: **24.01.92 CH 203/92**
**25.11.92 CH 3610/92**

(43) Veröffentlichungstag der Anmeldung:
**28.07.93 Patentblatt 93/30**

(84) Benannte Vertragsstaaten:
**AT BE CH DE DK ES FR GB IT LI NL**

(71) Anmelder: **F. HOFFMANN-LA ROCHE AG**
**Grenzacherstrasse 124**
**CH-4002 Basel(CH)**

(72) Erfinder: **Pauling, Horst**
**39 Ruchholzstrasse**
**CH-4103 Bottmingen(CH)**
Erfinder: **Wehrli, Christof**
**1 In den Gärten**
**CH-4108 Witterswil(CH)**

(74) Vertreter: **Urech, Peter, Dr. et al**
**Grenzacherstrasse 124 Postfach 3255**
**CH-4002 Basel (CH)**

(54) **Gamma-Lactonderivate als Antioxydantien.**

(57) Die Erfindung betrifft neue Antioxydantien, nämlich die - durch die in der Formel I vorkommende Wellenlinie angedeuteten - [R] bzw. [S] Verbindungen der Formel

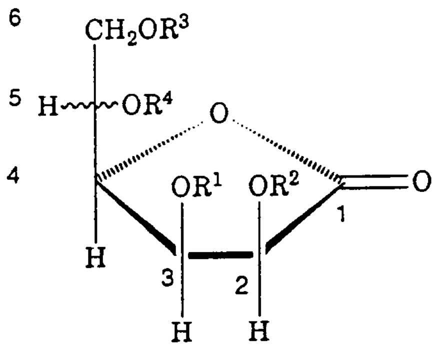

I

worin $R^1$ zusammen mit $R^2$ den Rest -S(O)- und $R^3$ und $R^4$ Wasserstoff, oder aber $R^3$ zusammen mit $R^4$ den Rest -S(O)-und $R^1$ und $R^2$ Wasserstoff darstellen.

Die Formel I soll insbesondere umfassen:
5,6-Sulfinyl-D- oder L-gulono-γ-lacton,
5,6-Sulfinyl-D-oder L-mannono-γ-lacton,
2,3-Sulfinyl-L-gulono-γ-lacton,
2,3-Sulfinyl-D-mannono-γ-lacton.

EP 0 552 651 A2

Die Erfindung betrifft neue Antioxydantien, nämlich die - durch die in der Formel I vorkommende Wellenlinie angedeuteten - [R] bzw. [S] Verbindungen der Formel

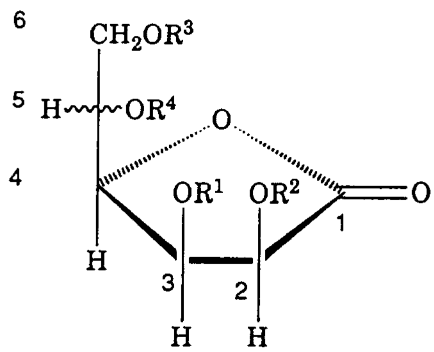

I

worin $R^1$ zusammen mit $R^2$ den Rest -S(O)- und $R^3$ und $R^4$ Wasserstoff, oder aber $R^3$ zusammen mit $R^4$ den Rest -S(O)-und $R^1$ und $R^2$ Wasserstoff darstellen.

Die Formel I soll insbesondere umfassen:

5,6-Sulfinyl-D- oder L-gulono-γ-lacton,

5,6-Sulfinyl-Doder L-mannono-γ-lacton,

2,3-Sulfinyl-L-gulono-γ-lacton,

2,3-Sulfinyl-D-mannono-γ-lacton.

Was die Stellung 4 betrifft, sollen demgemäss also beide epiFormen umfasst werden.

Die Erfindung betrifft auch ein Verfahren zur Herstellung der Verbindungen I.

Dieses Verfahren ist dadurch gekennzeichnet, dass man L-Gulonsäure-γ-lacton oder das jeweils, auch bezügl. Stellung 4 oder 5 entsprechende Stereoisomere, also D-Gulonsäure oder L-Mannonsäure- bzw. D-Mannonsäure-γ-lacton, sulfinyliert.

Die Erfindung betrifft schliesslich die Verwendung der Verbindungen I als Antioxydantien sowie Kompositionen mit oxydationshemmenden Eigenschaften, die durch einen Gehalt an einer Verbindung I gekennzeichnet sind.

Die Sulfinylierung des L-Gulonsäure-γ-lactons bzw. seines Stereoisomeren erfolgt vorzugsweise unter Verwendung von Thionylchlorid als Sulfinylierungsmittel. Die Reaktionspartner werden zweckmässigerweise in ungefähr äquimolekularen Mengen, vorzugsweise in möglichst äquimolaren Mengen, zur Reaktion gebracht. Zudem erfolgt die Sulfinylierung zweckmässigerweise bei Temperaturen von ca. - 20 °C bis ca. 50 °C. Ein bevorzugter Bereich ist derjenige von ca. 0 °C bis ca. 30 °C.

Man arbeitet zweckmässigerweise unter Zuhilfenahme eines inerten Lösungsmittels, insbesondere eines polaren, aprotischen Lösungsmittels; Beispiele solcher Lösungsmittel sind acyclische Di- und Polyäther, wie Monoglyme (1,2-Dimethoxyäthan) und Diäthylenglykoldimethyläther; cyclische Mono- und Diäther, wie Tetrahydrofuran und Dioxan; Sulfolan (Tetrahydrothiophen-1,1-dioxid); Dimethylformamid; Dimethylacetamid; N-Methylpyrrolidon; und Acetonitril, und dergleichen.

Die neuen Verbindungen besitzen interessante oxydationshemmende Eigenschaften und können in einer breiten Palette von Substraten eingesetzt werden, z.B.:

- in Lebensmitteln [Wein (insbesondere für dessen Schwefelung), Speiseessig, Speiseöle und -fette, Bier, Frucht- und Gemüsesäfte, Sirup, Fleisch und Wurstwaren, Dörrobst, Trockengemüse, Nüsse, Obst- und Gemüsekonserven, Kartoffelprodukte, Brotaufstriche, Saucen, Mayonnaise, etc.]
- in Futtermitteln
- in Kesselspeisewasser
- in Mitteln zur Herstellung und Entwicklung von Filmen
- in Druckmitteln
- in Mitteln zur Herstellung und Behandlung von Textilien
- in Kunststoffen
- in Schmierölen und -fetten
- in Kautschuk.

Die zweckmässig eingesetzte Menge der Verbindungen I kann jeweils klein gehalten werden; sie entspricht vorzugsweise ungefähr der eingesetzten Menge anderer Antioxydantien des Sulfit-typs, beträgt also z.B. zweckmässigerweise etwa ca. 1-7000 ppm, insbesondere ca. 20 bis ca. 2000 ppm. Die bevorzugte Menge richtet sich nach dem Anwendungszweck. In der Lebensmittelindustrie beträgt diese bevorzugte

Menge beispielsweise:

| | |
|---|---|
| für Wein: | ca. 50-300 ppm |
| für Dörrfrüchte: | ca. 1000-7000 ppm |
| für Frucht- und Gemüsesäfte: | ca. 80-300 ppm |

Die neuen Verbindungen I können als solche dem zu schützenden Substrat zugegeben werden, wo zweckdienlich, aber auch verdünnt, z.B. als Lösung, insbesondere als wässrige oder als alkoholische Lösung.

Bei höheren Konzentrationen (≧ 2 mg/ml Substrat) wirken die Verbindungen I zudem bakteriologisch und fungizid.

Beispiel

In einem 2 l-Vierhalssulfierkolben mit Thermometer, Tropftrichter und Plattenrührer werden unter Schutzgasatmosphäre 178,1 g L-Gulonsäure-$\gamma$-lacton (1,0 Mol), 400 ml Glaskugeln (d = 3 mm) in 1000 ml Tetrahydrofuran vorgelegt und unter Rühren in einem Eisbad auf ca. 0 °C abgekühlt. Dazu werden 72,6 ml Thionylchlorid (1,0 Mol) gegeben und die Suspension wird 1 Stunde bei 0 °C kräftig gerührt (600 rpm). Das Kühlbad wird entfernt und das Gemisch 15 Stunden bei Raumtemperatur gerührt. Die Glaskugeln werden über eine Glasschlitznutsche ohne Filter abgetrennt und mit THF nachgewaschen. Die Suspension wird auf ein Gemisch von 500 g Eis, 300 g Wasser und 1000 ml Essigester gegossen. Zur Neutralisation der Salzsäure werden portionenweise 162 g Natriumbicarbonat (1,93 Mol) unter Rühren zugegeben. Der pH-Wert wird durch Zugabe von weiterem Natriumbicarbonat auf pH = 5 gestellt. Das Produkt wird in 4 Scheidetrichtem mit 4 x je 500 ml Essigester extrahiert. Die organischen Phasen werden mit 3 x je 100 ml Wasser gewaschen, vereinigt und über 250 g Natriumsulfat während 1 Stunde getrocknet. Das Natriumsulfat wird abfiltriert und das Filtrat in einem 2 l-Rundkolben am Rollverdampfer (Badtemperatur = 50 °C) im Wasserstrahlvakuum eingeengt. Der Rückstand wird in 500 ml Aceton gelöst. Dazu werden 550 ml Essigester gegeben, das Aceton am Rollverdampfer unter Vakuum abdestilliert, danach wird 18 Stunden bei 0 °C kristallisiert. Das Produkt wird abgenutscht und mit 50 ml Essigester eiskalt nachgewaschen. Der Filterkuchen wird 5 Stunden bei 50 °C im Wasserstrahlvakuum bis zur Gewichtskonstanz getrocknet. Man erhält 91,5 g 5,6-Sulfinyl-L-gulono-$\gamma$-lacton (41% d.Th), Smp. 134,7 °C,

$$[\alpha]^{20}_{436} = (1\% \text{ in } H_2O) = +197°.$$

Weil das Produkt 5,6-[R/S]-Sulfinyl-L-gulono-$\gamma$-lacton ein Gemisch der beiden diastereomeren Sulfite ist (R:S = ca. 1:1), variieren Schmelzpunkt und Drehwert je nach Anteil der beiden Diastereomeren.

Aus der Mutterlauge kann das 2,3-Sulfinyl-L-gulono-$\gamma$-lacton gewonnen werden.

Die restlichen Isomeren der Formel I können analog erhalten werden.

## Patentansprüche

**1.** Verbindungen der Formel

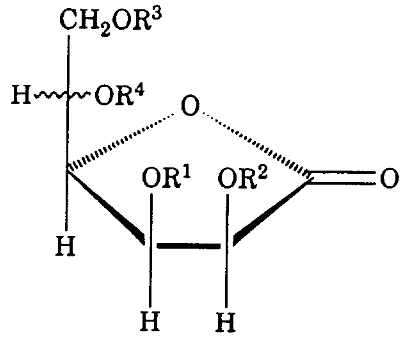

I

worin $R^1$ Zusammen mit $R^2$ den Rest -S(O)- und $R^3$ und $R^4$ Wasserstoff, oder aber $R^3$ Zusammen mit $R^4$ den Rest -S(O)-und $R^1$ und $R^2$ Wasserstoff darstellen.

**2.** 5,6-Sulfinyl-L-gulono-γ-lacton, 5,6-Sulfinyl-D-gulono-γ-lacton, 5,6-Sulfinyl-L-mannono-γ-lacton oder 5,6-Sulfinyl-D-mannono-γ-lacton.

**3.** 2,3-Sulfinyl-L-gulono-γ-lacton.

**4.** Verfahren zur Herstellung der Verbindungen der Formel

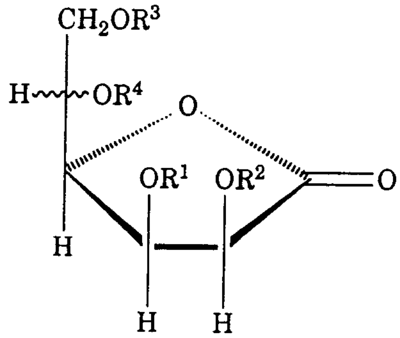

I

worin $R^1$ zusammen mit $R^2$ den Rest -S(O)- und $R^3$ und $R^4$ Wasserstoff, oder aber $R^3$ Zusammen mit $R^4$ den Rest -S(O)-und $R^1$ und $R^2$ Wasserstoff darstellen,
dadurch gekennzeichnet, dass man L-Gulono-γ-lacton oder ein entsprechendes Stereoisomeres hiervon sulfinyliert.

**5.** Verfahren nach Anspruch 4, dadurch gekennzeichnet, dass man L-Gulono-γ-lacton zu 5,6-Sulfinyl-L-gulono-γ-lacton sulfinyliert.

**6.** Verfahren nach Anspruch 4 oder 5, dadurch gekennzeichnet, dass man mittels Thionylchlorid sulfinyliert.

**7.** Komposition mit oxydationshemmenden Eigenschaften, gekennzeichnet durch einen Gehalt an einer Verbindung der Formel

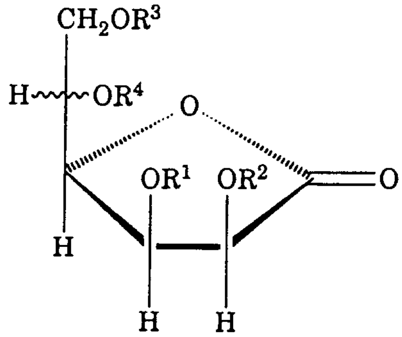

I

worin $R^1$ zusammen mit $R^2$ den Rest -S(O)- und $R^3$ und $R^4$ Wasserstoff, oder aber $R^3$ zusammen mit $R^4$ den Rest -S(O)-und $R^1$ und $R^2$ Wasserstoff darstellen,
oder einem Stereoisomeren davon.

**8.** Komposition nach Anspruch 7, gekennzeichnet durch einen Gehalt an 5,6-Sulfinyl-L-gulono-γ-lacton.

**9.** Verwendung der Verbindungen der Formel

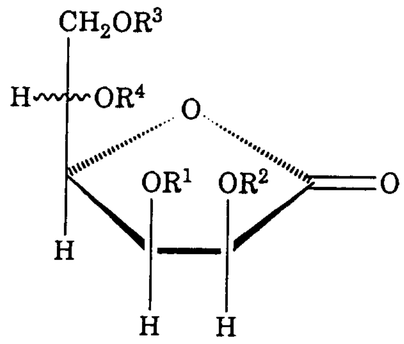

I

worin $R^1$ zusammen mit $R^2$ den Rest -S(O)- und $R^3$ und $R^4$ Wasserstoff, oder aber $R^3$ zusammen mit $R^4$ den Rest -S(O)-und $R^1$ und $R^2$ Wasserstoff darstellen, als Antioxydantien.

**10.** Verwendung von 5,6-Sulfinyl-L-gulono-$\gamma$-lacton als Antioxydans.